# FASCICULE DE BREVET EUROPEEN

(11) **EP 0 459 853 B1**
(45) Date de publication et mention de la délivrance du brevet: **01.02.1995**
(21) Numéro de dépôt: 91401237.2
(22) Date de dépôt: 14.05.1991
(51) Int. Cl.: A61B 8/08, G01S 15/89, G01S 7/52

(54) **Procédé d'acquisition d'images d'échographie**
Verfahren zur Erfassung von Echographie-Bildern
Acquisition method of echography images

(30) Priorité: 29.05.1990 FR 9006633
(43) Date de publication de la demande: 04.12.1991
(73) Titulaire: Touboul, Pierre-Jean, F-75008 Paris (FR); SOCIETE TRAITEMENT SYNTHESE IMAGE (T.S.I.) S.a.r.l., 92365 Meudon La Foret (FR)
(72) Inventeur: Touboul, Pierre-Jean, F-75008 Paris (FR); Tan, Siv-Chéng, F-94000 Saint-Maur (FR)
(74) Mandataire: Rodhain, Claude

(56) Documents cités:
- FR-A- 2 545 349
- PATENT ABSTRACTS OF JAPAN vol. 13, no. 313 (C-618)(3661) 17 Juillet 1989 & JP-A-01 097 441 (HITACHI) 14 Avril 1989
- PATENT ABSTRACTS OF JAPAN vol. 13, no. 394 (C-631)(3742) 31 Août 1989 & JP-A-01 139 044 (YOKOGAWA MEDICAL SYSTEMS) 31 Mai 1989
- PATENT ABSTRACTS OF JAPAN vol. 13, no. 372 (C-627)(3720) 17 Août 1989& JP-A-01 126 953 (HITACHI ) 19 Mai 1989

## Description

La présente invention a pour objet un procédé d'acquisition d'images d'échographie, en particulier d'images médicales d'échographie. Le but de l'invention est de permettre la comparaison d'images d'échographie acquises à différentes périodes de la vie d'un patient afin de déterminer si des pathologies qu'une première d'entre elles a révélé ont évolué au cours du temps.

On connaît le principe d'acquisition des images d'échographie. Celui-ci met en oeuvre un appareil échographe comportant une sonde munie de transducteurs piézo-électriques. L'appareil comporte aussi des circuits de commande pour faire émettre et pour recevoir des vibrations acoustiques par cette sonde. Pour obtenir une image d'une partie du corps d'un patient, on place la sonde contre la peau de ce patient à proximité de l'endroit de cette partie. Les vibrations acoustiques émises par la sonde sont réfléchies par les tissus de la partie examinée du corps, et viennent en retour exciter la sonde. Celle-ci, en réception, transforme les vibrations acoustiques reçues en des signaux électriques qui sont traités. Le traitement essentiel consiste à produire une image d'une coupe, une tomographie, du corps à l'endroit de la partie examinée.

Pour produire une image on fait balayer par des ondes, au cours d'une scrutation, la coupe à imager. Le balayage à deux dimensions dans la coupe par les ondes acoustiques est obtenu pour une direction de tir donnée, en établissant la relation entre les signaux reçus et le temps qui correspond à leurs réception. En effet, compte tenu de la vitesse de propagation des ondes acoustiques dans les milieux (dans les tissus humains, du fait de la grande teneur en eau de ces tissus, la vitesse de propagation est de l'ordre de 1500 mètres par seconde), les signaux réapparaissent sur la sonde d'autant plus tardivement après leurs réflexion qu'ils proviennent de zones profondes du corps. Normalement, ces signaux provenant de parties profondes sont atténués. On sait cependant compenser cette atténuation en provoquant une amplification correspondante dont le coefficient d'amplification varie avec le temps.

Pour balayer dans l'autre direction, on change tout simplement la direction de tir. Il existe plusieurs types de balayage de directions de tir. Ceux-ci sont soit des balayages dits latéraux, dans lesquels la direction de tir se déplace parallèlement à elle-même, soit des balayages dits angulaires dans lesquels les directions de tir couvrent une portion de cercle.

On sait représenter en temps réel, sur un moniteur de visualisation, les images produites par une sonde en provoquant par ailleurs un balayage électronique des directions de tir en synchronisation, ou en correspondance, avec le balayage vertical du moniteur de visualisation. Dans ce but on pilote le signal vidéo du moniteur par l'amplitude du signal acoustique détecté, démodulé et filtré.

Par ailleurs, un échographe est normalement muni de moyens pour stocker les images. Dans ce but, plutôt que d'utiliser une présentation analogique des signaux acoustiques reçus, on les numérise et on en stocke une version numérisée. Il est alors possible, au moyen d'une mémoire d'images en relation avec un moniteur de visualisation, de visualiser des images numérisées plutôt que des images analogiques elles-mêmes. Les matériels modernes sont actuellement munis de procédés de numérisation des signaux analogiques produits par la sonde tellement rapides que, même avec une mémoire d'images numérisées, on obtient une visualisation en temps réel. Le moniteur de visualisation montre l'image contenue dans la mémoire d'images. Celle-ci est en permanence rafraîchie par une nouvelle image produite par une nouvelle scrutation de la sonde.

Les images produites permettent au praticien de déceler la présence de pathologies particulières. Notamment dans les vaisseaux du cou, on cherche à déceler des lésions où l'apparence de plaques d'athéromes. On notera que ces plaques d'athéromes sont d'autant plus brillantes dans les images qu'elles correspondent à une transformation lipidefibrosecalcification de plus en plus avancée. En effet, leur densité augmente leur coefficient de réflexion. Les plaques d'athéromes, réduisant la souplesse des vaisseaux, ralentissent le débit de sang et peuvent provoquer à terme des lésions du cerveau par défaut d'irrigation. Il importe donc d'en surveiller l'évolution. Dans ce but, chez un patient on acquiert une image d'une région susceptible de contenir une telle plaque, ou la contenant déjà. Ultérieurement, par exemple quelques mois plus tard, on reprend une même image de la même région du corps. On compare alors les deux images pour essayer d'évaluer l'évolution, ou l'absence d'évolution de la pathologie en question.

Cette comparaison est difficile à mettre en oeuvre. En effet, elle est sujette aux variations des conditions d'acquisition. Une tentative de résoudre ce problème de comparaison a été présentée, en 1983, dans la revue Radiology Volume 148, N°2 Page 533-537 par MM David H.BLANDENHORN et Al, dans un article ayant pour titre "Common carotid artery contours reconstructed in three dimensions from parralel ultrasonic images". Dans la technique préconisée, il est proposé de rendre la sonde solidaire d'une potence motorisée susceptible d'imprimer à cette sonde une avance calibrée, de manière à acquérir des tranches parallèles aux autres. De cette façon, on est conduit à une reconstruction à trois dimensions d'une partie concernée du corps du patient. On pourrait imaginer de procéder ultérieurement, six mois plus tard, sur le même patient à une nouvelle expérimentation similaire. On pourrait alors tenter de retrouver dans les volumes reconstruits des tomographies correspondantes dans le but d'évaluer une quelconque évolution. Outre la complexité des matériels qu'il faut mettre en oeuvre, notamment pour produire une avance calibrée de la sonde, cette technique ne permettrait pas de donner des résultats probants parce que les images sont influencées par l'énergie acoustique mise en oeuvre à chaque fois. Les images doivent être calibrées. Or, il n'existe pas dans la théorie de l'absorption acoustique dans les tissus, de moyens de normalisation de la puissance des émetteurs acoustiques. L'expérimentateur souffre alors des dérives en puissance des appareils qu'il utilise. Le niveau de brillance est alors influencé par la puissance mise en jeu. De plus, le caractère non linéaire de l'absorption ne permet pas d'effectuer une normalisation.

Selon une autre publication, intitulée "Evaluation of a scoring system for extrancranial carotid atherosclerosis extent whit B-mode ultrasound" due à MM John R. CROUSE et Al paru dans la revue STROKE - Volume 17, N°2 Mars-Avril 1986 page 270 à 275, des mesures de sévérité, c'est à dire de l'épaisseur de la plus grande plaque d'athéromes détectées chez un patient, ont été effectuées à différentes périodes sur un lot de patients. Il en a été déduit d'une part, qu'entre la première et la deuxième campagne de mesures de sévérité, les corrélations étaient faibles, compte tenu qu'on s'intéressait à la lésion la plus sévère. Par contre, avec une mesure dite d'extension, qui se référait à la somme des épaisseurs axiales de toutes les obstructions locales, les corrélations étaient meilleures. Bien que les conclusions de cet article aient amené à des considérations statistiques utiles, elles ne peuvent avoir, pour l'examen de chaque individu, qu'un seul enseignement : la difficulté de comparer des images acquises à une période aux images acquises à une autre période rend l'évaluation de l'évolution d'une pathologie hasardeuse.

Il est connu, notamment en angiographie par rayons X, des procédés de recalage d'images dans lesquels, pour effectuer la soustraction d'images, on transforme une des deux images. On en corrige la distorsion pour qu'elle se redresse et vienne, par superposition, correspondre à l'autre image. On note cependant qu'une telle technique nécessite d'une part des moyens de calcul très puissants (elle conduit à des tailles mémoires importantes), et d'autre part n'est pas transposable au domaine de l'échographie où la permanence de l'orientation de la sonde par rapport au corps du patient n'est pas aussi bien assurée que celle de l'orientation d'un tube à rayons X par rapport au corps du patient dans une application d'angiographie radiologique.

L'invention a pour but de remédier à ces inconvénients en proposant une méthode complètement différente mais qui soit à la portée directement de tous les praticiens utilisateurs des échographes existant déjà sur le marché. L'invention procure non seulement des résultats statistiques applicables à une population d'individus, mais aussi des résultats concernant chaque individu. L'invention part de la constatation suivante, différente de ce qui se pratiquait dans l'état de la technique, que les praticiens eux-mêmes sont capables, avec une certaine expérience, de voir en temps réel les structures qu'ils veulent montrer. On admet par exemple, qu'au bout de 300 ou 400 échographies pratiquées sur des patients différents, le praticien devient vraiment habile dans la manipulation de sa sonde pour faire apparaître ce qu'il cherche à voir, en particulier pour l'acquisition d'une première image, les lésions et les plaques d'athéromes dans les artères carotides du cou.

Plutôt que de faire subir alors un traitement de correction de distorsion à des images acquises ultérieurement par ces praticiens, il a été imaginé d'imposer à ces praticiens, pour l'acquisition des deuxièmes images révélatrices, une contrainte supplémentaire. Cette contrainte supplémentaire consiste à leur faire placer la sonde, pour la deuxième image, exactement dans la même position que celle qui cette sonde occupait au moment de l'acquisition de l'image précédente. Pour matérialiser cette contrainte, on produit alors, en toile de fond de l'écran de visualisation en temps réel des images acquises pour la deuxième image, les contours des tissus étudiés et acquis lors de la première image.

Le praticien est alors amené, d'une part à devoir montrer une image représentative de la lésion, et d'autre part à affiner la présentation de cette lésion, en manipulant la sonde, en tournant la main, de telle manière que la deuxième image se mette en coïncidence le plus exactement possible avec l'image de fond. On dit alors qu'il met la deuxième image dedans. L'image de fond peut être par ailleurs représentée en une surbrillance. On s'est rendu compte alors qu'il suffisait, à un des inventeurs de la présente invention, d'une cinquantaine seulement d'examens supplémentaires pour arriver à obtenir une dextérité suffisante pour qu'une deuxième image puisse être considérée comme acquise sensiblement avec des mêmes conditions d'inclinaison et d'incidence de la sonde que celles qui prévalaient pour la première image.

Par ailleurs, pour résoudre le problème qui pourrait subvenir du fait des dérives en puissance des appareils d'échographie, on présente, à un endroit de l'image visualisée une information sur la densité des tissus. Cette information est donnée par la mesure de la luminosité moyenne des éléments d'images, des pixels, contenus dans un même dessin (un cercle) dans la première image puis, dans un même dessin au même endroit, dans la nouvelle image. Au moyen d'un bouton de réglage du gain, le praticien opérateur peut alors ajuster la puissance et/ou la forme de l'impulsion d'excitation acoustique de manière à ce que les mesures deviennent compatibles. Quand celles-ci sont compatibles, il est possible d'évaluer l'évolution de la luminosité de la plaque d'athérome surveillée et d'en déduire si celle-ci s'est ou non plus calcifiée depuis le dernier examen.

Il est estimé que cette validation visuelle, est encore subjective, puisqu'elle passe par une appréciation humaine de la ressemblance mesurée. En effet, elle est liée au discernement du praticien et elle n'est pas le résultat d'un traitement objectif, effectué par une machine de traitement, en fonction d'un processus automatique. Cette validation a cependant un certain avantage par rapport à ce qui se pratiquait auparavant. Il est estimé en particulier que l'acquisition de la deuxième image peut être pratiquée par un praticien différent de celui qui a pratiqué la première image. Par ailleurs, le fait de présenter un contour de la première image apporte de meilleurs résultats que si on avait tenté de présenter toute la première image.

En effet, on connaît des systèmes dans lesquels on divise l'écran de visualisation en temps réel en deux parties, une partie, par exemple la gauche, étant destinée à montrer la première image tandis que l'autre partie, la droite, étant destinée à montrer la nouvelle image en cours d'acquisition. Dans de tels cas, compte tenu de l'existence d'une lésion ou de tout autre pathologie dans la première image, on constate que le praticien cherche instinctivement à présenter une deuxième image dans laquelle cette lésion a une apparence plus grande, égale ou plus faible, selon que ce praticien est poussé par le désir intime de montrer respectivement une aggravation, une stagnation, ou une régression de cette pathologie. Dans ce cas, la contrainte subjective est trop forte et les mesures peu fiables.

Par contre, en ne montrant que le contour de la première image, et éventuellement aussi les mesures de densités, le praticien doit acquérir une image qui concorde le plus possible avec l'image ancienne sans savoir ce qui l'attend quand il comparera par la suite la deuxième image à la première image vraie (et non plus seulement à ses contours). Sur cette nouvelle image acquise, il compare alors, et seulement alors, les endroits pathologiques avec la première image. La démarche est différente en ce sens que l'importance de la pathologie mesurée précédemment n'est pas révélée en temps réel au praticien, mais qu'elle lui apparaît ensuite comme un résultat objectif de comparaison. En effet, comme elle n'est pas présente dans l'image des contours montrés, elle ne peut pas être incitative.

L'invention permet ainsi une reproductibilité morphologique et densitométrique des images basée sur les aptitudes d'intégration mentale du praticien qui manipule la sonde. Le coût de cette aptitude supplémentaire, on a pu le constater, est limité à l'expérimentation d'une cinquantaine de cas. Au-delà, la dextérité est acquise. En particulier, avec l'expérience, on a constaté que les contours choisis par le praticien sont de plus en plus rapidement déterminés dans la première image : c'est à dire même avec un faible nombre de points. On a cependant pu constater que la reproductibilité n'était pas parfaite. Par contre, elle est nettement supérieure à ce qu'on était susceptible de faire auparavant. Par exemple, on a pu relever des évolutions de lésions de l'ordre de 5% ce qui n'était pas envisageable avec les techniques anciennes.

Dans un perfectionnement, on utilise le fait que les échographes modernes sont munis d'une mémoire d'images et de moyens de stocker des images numérisées. Dans ce cas, au fur et à mesure que le praticien manipule la sonde, à proximité de l'orientation et de l'inclinaison auxquelles il doit aboutir, on mémorise les différentes images acquises en temps réel. Par la suite, en temps différé, on peut choisir parmi la série d'images acquises laquelle est la meilleure, c'est à dire laquelle se rapproche le plus par superposition des contours indiqués sur l'écran et relatifs à la première image.

On s'est par ailleurs rendu compte que cette technique était particulièrement intéressante lors de l'étude des phénomènes cycliques. Elle est donc particulièrement intéressante pour l'étude du phénomène d'écoulement du sang dans les artères. Ceci était en soi une autre limitation aux techniques anciennes.

L'invention a donc pour objet un procédé d'acquisition d'images d'échographie médicale obtenues avec un appareil d'échographie, cet appareil étant muni d'un écran de visualisation en temps réel des images produites, dans lequel :
- on acquiert avec cet appareil une première image d'une région du corps d'un patient à examiner, selon une incidence et une inclinaison particulières d'une sonde d'échographie de cet appareil,
- un praticien acquiert ultérieurement une deuxième image de la même région du corps du même patient, selon les mêmes incidences et inclinaisons de la sonde du même appareil,
- et on compare les deux images acquises pour évaluer l'évolution du corps de ce patient entre la date de la première image et celle de la deuxième image,
   caractérisée en ce que
- on assiste le praticien au cours de l'acquisition de la deuxième image, en produisant, sur l'écran de visualisation en temps réel, une image de contours de structures présentes dans la première image,
- et en ce que ce praticien manipule la sonde de façon à ce que les structures de la deuxième image, visibles en temps réel, se superposent à l'image des contours déjà présents de la première image.

L'invention sera mieux comprise à la lecture de la description qui suit et à l'examen des figures qui l'accompagnent. Celles-ci ne sont données qu'à titre indicatif et nullement limitatif de l'invention.

Les figures montrent :
- figure 1 : une représentation schématique d'un appareil d'échographie pour mettre en oeuvre le procédé de l'invention,
- figure 2 : une représentation schématique du cou d'un patient,
- figure 3 : une image prise en inclinaison transverse du cou présenté sur la figure 2.

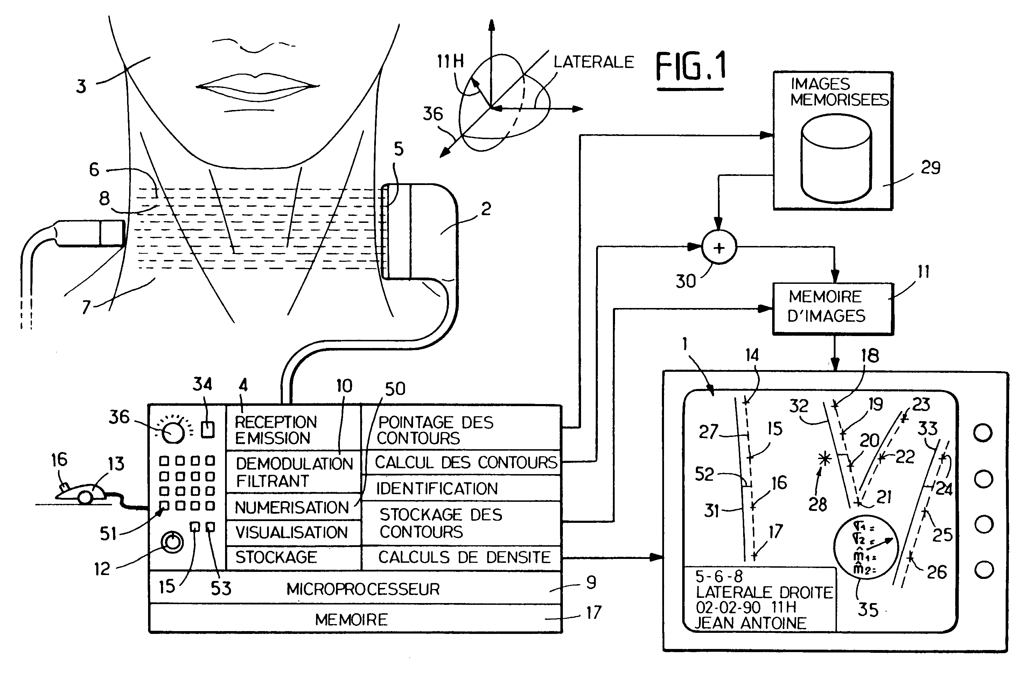

La figure 1 montre un dispositif d'échographie utilisable pour mettre en oeuvre le procédé de l'invention. Ce dispositif comporte un écran de visualisation 1 pour montrer en temps réel les images produites. Selon le procédé on acquiert des images à deux dates différentes. Le processus d'acquisition, hormis le perfectionnement de l'invention, est le même dans les deux cas. Le processus d'acquisition consiste d'une manière connue en soi à placer une sonde d'échographie 2 contre une région du corps d'un patient 3. Dans l'exemple représenté, la région examinée est le cou, et l'application décrite est une application d'artériographie. Cependant, il est envisageable d'utiliser l'invention à d'autres fins, notamment pour voir l'arbre vasculaire des membres inférieurs, supérieurs ou des organes situés dans le tronc du patient : l'estomac,le foie.

L'appareil d'échographie comporte classiquement des moyens d'émission réception 4 pour envoyer à la sonde 2 des signaux électriques. Les signaux électriques y sont transformés en signaux acoustiques par des transducteurs piézo-électriques 5 de la sonde 2. Les signaux électriques sont par exemple des impulsions de fréquence élevée (de l'ordre de 10 Mhz) et de durée courte. Ces signaux sont transmis, selon des procédés connus en eux mêmes, aux différents transducteurs 5 de manière à focaliser le tir dans des directions de tir 6. Dès que l'impulsions d'excitation est terminée, l'échographe bascule en réception notamment par l'action d'un multiplexeur. Les transducteurs piézo-électriques 5 traduisent en signaux électriques les excitations acoustiques qu'ils reçoivent par réflexion depuis le cou 7 du patient. Les signaux acoustiques réfléchis aboutissent aux transducteurs 6 de plus en plus tard en fonction de la distance séparant la zone dont ils proviennent de la sonde 2. Lorsqu'on considère que tous les signaux relatifs à une direction sont revenus, on tire dans une direction voisine, par exemple la direction 8, et on mesure les mêmes signaux acoustiques réfléchis.

L'organisation du balayage des directions de tir 6 et 8 de même que la réception des signaux acoustiques sont gérées par un microprocesseur 9. Ce microprocesseur 9 gère en correspondance les balayages ligne et les balayages colonne du moniteur de visualisation 1. Si le balayage de direction de tir n'est pas de type latéral, mais au contraire de type en éventail, la visualisation sur le moniteur 1 est organisée en conséquence.

Les signaux mesurés correspondent aux signaux démodulés et filtrés dans une opération 10. Comme cela a déjà été indiqué précédemment, plutôt que de visualiser comme signal vidéo un signal analogique démodulé filtré, on a de plus en plus recours à une numérisation en une opération 60 du signal analogique. Le signal numérique est alors transmis à une mémoire d'images 11 dont la lecture est gérée aussi par le microprocesseur 9. Au moment de la lecture, le microprocesseur prélève les informations contenues à chaque adresse des cellules d'une page mémoire de la mémoire d'image et les utilise comme signal de luminance et/ou de chrominance pour piloter en correspondance la luminosité d'un pixel sur le moniteur 1.

Il est connu, dans l'état de la technique, des systèmes pour pointer des lieux particuliers sur les images. Dans ce but, au moyen d'une boule de commande 12, ou éventuellement d'une souris 13, un praticien amène un index lumineux, par exemple une croix 14, à occuper une place sur l'écran. Pour provoquer ensuite l'enregistrement en mémoire de la place de cet index 14, au moment où cet index est placé, le praticien appuie sur un bouton de commande 15 pour la boule ou 16 pour la souris. Dans ce cas, le microprocesseur 9 enregistre, dans sa mémoire de travail 17 les coordonnées du lieu sur l'écran de l'index 14. Il associe aussi les coordonnées de ce lieu à une adresse dans une page mémoire de la mémoire d'image. Le praticien peut ensuite déplacer l'index pour que celui-ci vienne occuper une autre position 15. Les portions 14 et 15 sont situées sur un contour de la première image. Ainsi, de suite, les adresses des lieux des index 14 à 26 peuvent être enregistrées.

Il est par ailleurs déjà connu de provoquer l'affichage sur l'écran d'un segment droit reliant deux index entre eux. Par exemple, on peut utiliser un tel procédé pour venir enregistrer dans la mémoire 17, et par ailleurs faire apparaître en surimpression lumineuse sur l'écran 1, le segment 27 situé entre les index 14 et 15. Par ailleurs, il est connu par des routines de type SPLINE ... de faire passer une courbe du deuxième degré (plutôt qu'une droite) entre un certain nombre d'index prédéterminés : par exemple les index 14 à 17, ou 18 à 21, ou 21 à 23, ou 24 à 26. Un exemple d'un tel programme est donné en annexe. Dans ce cas, par le clavier 51 de l'appareil on indique les débuts et fin de segment de courbe.

Dans l'invention, compte tenu de l'acquisition d'une première image montrant une plaque d'athérome 28, on peut par ailleurs pointer cette plaque d'athérome par un index spécial, ici en forme d'astérisque, de manière à signaler la présence d'une lésion. Ceci permet ultérieurement au praticien de vérifier l'existence dans les nouvelles images de la même lésion. Une fois que les contours 14 à 26 et les segments tels que 27 ont été élaborés, ces segments plutôt que de rester dans la mémoire 17 sont chargés dans une image des contours contenue dans une mémoire d'images mémorisées 29. Cette acquisition des contours s'exécute de préférence sur une première image visualisée, maintenue fixe sur l'écran 1.

Lors de l'acquisition de la deuxième image, on envoie les images produites au cours de cette deuxième acquisition, en temps réel, directement sur le moniteur 1, à travers le passage néanmoins de la mémoire d'image 11. La mémoire d'image comporte donc pour chaque adresse de pixel de l'écran 1, une information de luminosité correspondant à la valeur numérisée du signal acoustique détectée en temps réel. Au moyen d'un additionneur 30, on additionne alors pixel à pixel (c'est à dire avec les bonnes adresses) les informations lumineuses relatives aux contours de la première image qui ont été préalablement stockées dans la mémoire 29 d'images mémorisées. Autrement dit, au cours de la deuxième acquisition le praticien voit apparaître sur écran les contours 14 à 27 (ainsi que l'astérisque 28). Il doit alors tenter, en manipulant la sonde 2, de faire en sorte que l'image qu'il produit en temps réel sur l'écran, et qui est symbolisée ici par les lignes fines 31 à 33, viennent se superposer exactement sur les contours représentés par les croix et les tirets. Cela peut consister dans un cas favorable à faire subir à l'image temps réel le déplacement indiqué par la flèche 52.

C'est pour aboutir à cette habileté de manoeuvre qu'il faut environ une cinquantaine d'expériences. Lorsque le praticien sent qu'il approche de la concordance, il peut provoquer, en appuyant sur un bouton de commande 34, la mémorisation dans la mémoire 17 des images produites par la sonde 2 et correspondant à des scrutations successives du balayage acoustique. Ou bien le praticien est très expérimenté, et il est capable d'aboutir très rapidement à la concordance stable, ou bien il est moins expérimenté et il y aboutit d'une manière fugitive. Dans les deux cas, avec la mémorisation d'une série d'images il est possible, quelques instants plus tard en temps différé, de réexaminer les images acquises lors de la deuxième expérimentation et de choisir celle parmi ces images pour laquelle la concordance semble être la meilleure.

Pour résoudre le problème de gain, il est prévu de réserver dans la première image un motif, dans un exemple un cercle tel que le cercle 35, dont on peut élaborer pour l'ensemble des pixels qu'il contient la valeur m et l'écart type σ de la variation de luminosité. Le motif 35 est placé de préférence dans un endroit relativement stable dans l'image : là où le signal réfléchi et récupéré varie peu quelles que soient les orientations prises par la sonde. Pour faire la moyenne, il suffit d'additionner l'ensemble des valeurs d'informations contenues dans les cellules mémoires, contenu dans la mémoire d'images 11, et correspondant à l'intérieur du motif 36 et de diviser le résultat de l'addition par le nombre de pixels. L'écart type est calculé d'une manière similaire. De même qu'on a stocké comme image mémorisée la position des index 15 à 26 et la position des segments tels que 27, on peut stocker la position du motif 36 ainsi que les valeurs σ₁ et m₁ relatif à la première image.

Au cours de l'acquisition de la deuxième image, on peut montrer, respectivement chaque fois en dessous de la valeur correspondante, les valeurs σ₂ et m₂ relatives à la luminosité des pixels situés dans un même motif 35, placé au même endroit. En effet, ces calculs simples peuvent eux aussi être effectués par des opérateurs qui reçoivent en permanence les informations de luminosité en provenance des cellules mémoires concernées. Au moyen d'un bouton de commande de gain 36, le praticien est alors capable de faire varier le gain de l'échographe : c'est à dire la puissance des signaux acoustiques émis par la sonde 5. De cette manière les valeurs de luminosité tendent à devenir les mêmes entre la première et la deuxième images. On souhaite qu'elles soient les mêmes ou le plus proche possible l'une de l'autre. Les valeurs de luminosité représentent en fait la densité du matériau rencontré. En effet, plus le matériau rencontré est dense, plus les signaux réfléchis vont être nombreux et plus la valeur moyenne m sera élevée. D'une manière préférée, on place le cercle 35 à un endroit où en outre la moyenne est faible.

Afin d'aider le praticien encore un peu plus on provoquera de même, en surimpression, une identification indiquant d'une part le nom du patient, d'autre part, la date de la première acquisition, et enfin l'orientation, 11H, et l'incidence, LATERALE DROITE, dans laquelle se trouvait la sonde au moment de la première acquisition. L'orientation correspond à une orientation par rapport à la vertical et montre dans un repérage horaire ou anti-horaire la position de la sonde dans le plan vertical. Par ailleurs, l'incidence montre, par rapport à un axe 36 passant à travers le dos du patient, l'orientation antérieure, postérieure, latérale ou intermédiaire entre ces directions, de la sonde. Par ailleurs, on peut également donner l'indication des segments, 5-6-8, d'artères carotides visualisés.

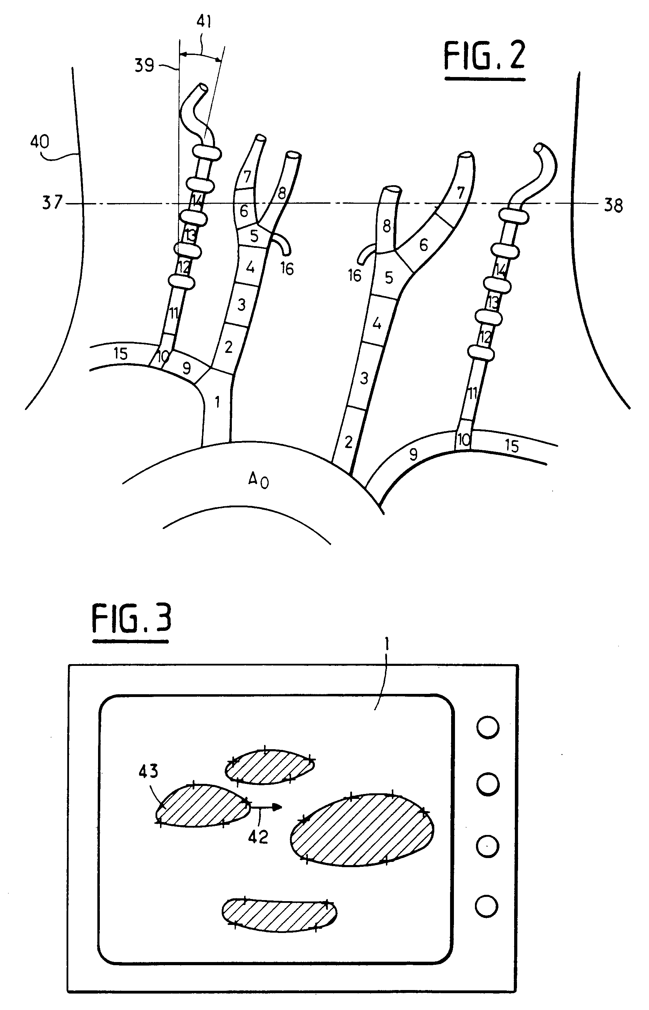

La figure 2 montre d'une manière schématique l'anatomie des segments d'artères carotides.

Les numéros des segments représentent dans l'ordre
1 - tronc artériel brachiocéphalique,
2 - carotide primitive basse,
3 - carotide primitive moyenne,
4 - carotide primitive haute,
5 - bifurcation carotidienne,
6 - bulbe carotidien,
7 - carotide interne sous-bulbaire,
8 - carotide externe
9 - sous clavière prévertébrale,
10 - sous clavière et origine de la vertébrale,
11 - vertébrale prétransverse,
12 - vertébrale intertransverse premier segment,
13 - vertébrale intertransverse deuxième segment,
14 - vertébrale intertransverse troisième segment,
15 - sous clavière post vertébrale.

Au moment de l'acquisition de la deuxième image ces indications montrent déjà au praticien où et approximativement comment il doit disposer sa sonde. Ces indications sont en gros suffisantes pour que la distance 52 dans le cas le plus simple où la superposition des images est obtenue par une translation de l'image temps réel ne soit pas supérieure à la dimension de l'écran.

La figure 3 montre l'image des contours résultant d'une première acquisition d'une coupe transverse, orientée par exemple selon l'orientation 37-38 indiquée sur la figure 2. Il pourrait sembler que les contours représentés soient insuffisants pour permettre de déterminer à quelle hauteur la première image a été faite. On s'est rendu compte cependant qu'une telle situation ne se produisait pas parce que, d'une part les vaisseaux examinés n'ont pas des diamètres constants quelle que soit leur hauteur et que, d'autre part, ils ne sont pas orientés verticalement. Par rapport à la verticale 39 (parallèle au bord 40 du cou) contre lequel glisse la sonde 2 on peut déterminer que tous les vaisseaux forment un certain angle tel que 41. Si on se déplace alors en hauteur, vers le haut, cet angle 41 provoque un déplacement orienté dans le sens de la flèche 42, des images des structures concernées. Autrement dit, compte tenu d'une image 1 acquise et représentée, on est capable d'aboutir assez rapidement à la représentation de la deuxième image. On constate dans ce cas qu'il y a intérêt pour les coupes à avoir un nombre de segments ou de contours un peu plus élevé que pour les images de coupes non transverses. Autant, il faut pour ces dernières en moyenne une dizaine d'index pour effectuer le repérage, il en faut environ le double pour effectuer le repérage des images de coupes transverses.

Bien entendu, l'invention n'est pas limitée à l'exemple de réalisation ci-dessus décrit et on pourra prévoir d'autres variantes sans pour cela sortir du cadre de l'invention.

## Revendications

1. Procédé d'acquisition d'images d'échographie médicale obtenues avec un appareil d'échographie, cet appareil étant muni d'un écran (1) de visualisation en temps réel des images produites, dans lequel
- on acquiert (2-10) avec cet appareil une première image d'une région du corps d'un patient (3) à examiner, selon une incidence et une inclinaison (11H) particulière d'une sonde d'échographie de cet appareil,
- un praticien acquiert ultèrieurement une deuxième image de la même région du corps du même patient, selon les mêmes incidences et inclinaisons de la sonde du même appareil,
- et on compare les deux images acquises pour évaluer l'évolution du corps de ce patient entre la date de la première image et celle de la deuxième image,
caractérisé en ce que
- on assiste le praticien, au cours de l'acquisition de la deuxième image, en produisant, sur l'écran de visualisation en temps réel, une image (14-27, 43) de contours de structures présentes dans la première image,
- et en ce que ce praticien manipule la sonde de façon à ce que les contours de la deuxième image, visibles en temps réels, se superposent à l'image des contours déjà présents de la première image.

2. Procédé selon la revendication 1, caractérisé en ce que
- on produit, sur l'écran de visualisation en temps réel, des informations (35, m, σ) de densité de tissus du corps relatifs à la première images,
- et des informations de densité des mêmes tissus du corps relatifs à la deuxième image,
- et en ce que le praticien règle (36) son appareil pour que ces informations de densité soient compatibles.

3. Procédé selon l'une quelconque des revendications 1 ou 2, caractérisé en ce que
- on produit en outre, sur l'écran de visualisation en temps réel, des indications relatives à l'une quelconque des caractéristiques suivantes : incidence de la première image, inclinaison de la première image (11H), numéro d'un tronçon du corps de la première image, (non du patient, date de la première image).

4. Procédé selon l'une quelconque des revendications 1 à 3, caractérisé en ce que, pour produire l'image des contours de la première image,
- on mémorise une première image et on la visualise, en temps différé, sur l'écran de visualisation en temps réel,
- on pointe (13) sur cette première image mémorisée et visualisée des index (14), au moyen de boutons de commandes,
- et on exécute un traitement de tracé de courbe pour produire une courbe (27) qui relie, sur l'écran, ces index.

5. Procédé selon l'une quelconque des revendications 1 à 4, caractérisé en ce que, pour acquérir une bonne deuxième image,
- le praticien acquiert en temps réel plusieurs deuxièmes images successives, alors que la sonde est proche de la position de la première image et que l'échographe émet ces deuxièmes images à un rythme de scrutation qui lui est propre,
- on mémorise ces deuxièmes images (17), et
- on choisit, en temps différé, comme bonne parmi ces deuxièmes images, celle qui s'apparente le plus à la première image.

6. Procédé selon l'une quelconque des revendications 1 à 5, caractérisé en ce que, pour produire l'image des contours de la première image,
- on choisit la première image parmi une série d'images successives.

## Claims

1. A method for acquiring medical echography images obtained with an echography device, the device being provided with a screen (1) for displaying in real time the images produced in which
- a first image of a body area of a patient to be examined (3) is obtained (2-10) with this device, according to a particular angle of incidence and angle of inclination (11H) of an echography probe of this device,
- a practitioner subsequently obtains a second image of the same body area of the same patient, according to the same angles of incidence and inclination of the probe of the same device,
- and in which the two images obtained are compared to evaluate how the patient's body has developed between the date of the first image and that of the second image,
characterised in that
- while the second image is being obtained the practitioner is assisted by producing on the real time display screen an image (14-27, 43) of the shapes of structures present in the first image,
- and in that this practitioner moves the probe in such a way so as to superimpose the shapes of the second image, displayed in real time, on the image of the existing shapes of the first image.

2. A method according to Claim 1, characterised in that
- data (35, m, σ) on the weight of the body tissues relating to the first image,
- and data on the weight of the same body tissues relating to the second image are produced on the real time display screen,
- and in that the practitioner controls (36) his device so that these weight data are compatible.

3. A method according to one of Claims 1 or 2, characterised in that
- data relating to any one of the following characteristics are also produced on the real time display screen: the angle of incidence of the first image, the angle of inclination of the first image (11H), the number of a body section from the first image, (the name of the patient, the date of the first image).

4. A method according to any one of Claims 1 to 3, characterised in that to produce the image from the shapes of the first image,
- an initial image is recorded and displayed later on the real time display screen,
- control buttons are used to plot (13) points (14) on this initial image recorded and displayed,
- and an outline of a curve is processed to produce a curve (27) linking these points on the screen.

5. A method according to any one of Claims 1 to 4, characterised in that, to obtain a good second image
- while the probe is close to the position of the first image and the echograph is emitting these second images with a scanning rhythm which is appropriate thereto, the practitioner obtains several successive second images in real time,
- these second images (17) are recorded, and
- the image which appears to be closest to the first image is chosen later as being the best of the second images.

6. A method according to any one of Claims 1 to 5, characterised in that, to produce the image from the shapes of the first image,
- the first image among a series of successive images is chosen.

## Patentansprüche

1. Verfahren zur Erfassung von mit einem Echographie-Gerät erhaltenen medizinischen Echographie-Bildern, wobei das Gerät mit einem Bildschirm (1) zur Echtzeitdarstellung der erzeugten Bilder versehen ist, bei dem
- man mit dem Gerät ein erstes Bild eines Bereiches des Körpers eines zu untersuchenden Patienten (3) gemäß einem einer Echographie-Sonde des Geräts eigenen Anstellwinkel und einem Neigungswinkel (11H) erfaßt (2 - 10),
- ein Arzt später ein zweites Bild des gleichen Bereiches des Körpers des gleichen Patienten gemäß dem gleichen Anstellwinkel und Neigungswinkel der Sonde des gleichen Gerätes erfaßt,
- und man die beiden erfaßten Bilder zum Beurteilen der Entwicklung des Körpers des Patienten zwischen dem Zeitpunkt des ersten Bildes und dem des zweiten Bildes vergleicht,
dadurch gekennzeichnet, daß
- der Arzt im Verlauf des Erfassens des zweiten Bildes unterstützt wird, indem auf dem Bildschirm zur Echtzeitdarstellung ein Bild (14 - 27, 43) der Umrisse der im ersten Bild vorhandenen Strukturen erzeugt wird,
- und daß der Arzt die Sonde in einer Weise bedient, daß die in Echtzeit sichtbaren Umrisse des zweiten Bildes sich dem Bild der bereits vorhandenen Umrisse des ersten Bildes überlagern.

2. Verfahren nach Patentanspruch 1, dadurch gekennzeichnet, daß
- auf dem Bildschirm zur Echtzeitdarstellung auf das erste Bild bezogene Informationen (35, m, σ) bezüglich der Dichte des Körpergewebes,
- und auf das zweite Bild bezogene Informationen bezüglich der Dichte des selben Körpergewebes dargestellt werden,
- und daß der Arzt sein Gerät so regelt (36), damit diese Informationen bezüglich der Dichte miteinander vereinbar sind.

3. Verfahren nach einem der Patentansprüche 1 oder 2, dadurch gekennzeichnet, daß
- auf dem Bildschirm zur Echtzeitdarstellung unter anderem auf eines der folgenden Kennzeichen bezogene Anzeigen erzeugt werden: Anstellwinkel des ersten Bildes (11H), Nummer des Teilstückes des Körpers des ersten Bildes, (Name des Patienten, Datum des ersten Bildes).

4. Verfahren nach einem der Patentansprüche 1 bis 3, dadurch gekennzeichnet, daß man zum Erzeugen des Bildes der Umrisse des ersten Bildes
- ein erstes Bild speichert und zu einem späteren Zeitpunkt auf dem Bildschirm zur Echtzeitdarstellung darstellt,
- auf diesem ersten gespeicherten und dargestellten Bild Indizes (14) mittels Steuertasten einstellt (13),
- und zum Erzeugen einer diese Indizes auf dem Bildschirm verbindenden Kurve (27) eine Kurvenzeichnungs-Verarbeitung durchgeführt wird.

5. Verfahren nach einem der Patentansprüche 1 bis 4, dadurch gekennzeichnet, daß zum Erfassen eines guten zweiten Bildes
- der Arzt mehrere aufeinanderfolgende zweite Bilder in Echtzeit erfaßt, während die Sonde der Position des ersten Bildes nah ist, und daß der Echograph diese zweiten Bilder mit einem für ihn eigentümlichen Untersuchungstakt abgibt,
- man diese zweiten Bilder speichert (17), und
- man unter den zweiten Bildern zu einem späteren Zeitpunkt jenes als gut auswählt, das dem ersten Bild am ähnlichsten ist.

6. Verfahren nach einem der Patentansprüche 1 bis 5, dadurch gekennzeichnet, daß man zum Erzeugen des Bildes der Umrisse des ersten Bildes
- das erste Bild aus einer Serie aufeinanderfolgender Bilder auswählt.
